# EUROPÄISCHE PATENTSCHRIFT

(11) **EP 2 480 134 B1**
(45) Veröffentlichungstag und Bekanntmachung des Hinweises auf die Patenterteilung: **03.12.2014**
(21) Anmeldenummer: 10760918.2
(22) Anmeldetag: 17.09.2010
(51) Int. Cl.: A61B 5/055, A61B 19/00, A61B 6/00, A61B 6/04

(54) **VORRICHTUNG ZUR MAMMABIOPSIE**
EQUIPMENT FOR USE IN MAMMARY BIOPSY
DISPOSITIF DE BIOPSIE MAMMAIRE

(30) Priorität: 25.09.2009 DE 102009042741; 08.01.2010 DE 102010004236
(43) Veröffentlichungstag der Anmeldung: 01.08.2012
(73) Patentinhaber: Bergers, Diethard, 45133 Essen (DE); Kower, Oliver, 47249 Duisburg (DE); Lubnau, Jörg, 45525 Hattingen (DE); Tracht, Rudolf, 44265 Dortmund (DE)
(72) Erfinder: Bergers, Diethard, 45133 Essen (DE); Kower, Oliver, 47249 Duisburg (DE); Lubnau, Jörg, 45525 Hattingen (DE); Tracht, Rudolf, 44265 Dortmund (DE)
(74) Vertreter: Von Rohr Patentanwälte Partnerschaft mbB
(86) Internationale Anmeldenummer: PCT/EP2010/005715
(87) Internationale Veröffentlichungsnummer: WO 2011/035869

(56) Entgegenhaltungen:
- WO-A2-2006/030406
- DE-A1-102006 038 163
- US-A- 3 971 950
- US-A1- 2007 280 412
- US-A1- 2008 240 345
- US-A1- 2009 118 614
- US-A1- 2009 213 986

## Beschreibung

Die vorliegende Erfindung betrifft eine Vorrichtung zur Mammauntersuchung, insbesondere Biopsie, gemäß dem Oberbegriff des Anspruchs 1.

Die vorliegende Erfindung bezieht sich insbesondere auf eine Vorrichtung zur zweidimensionalen Positionierung eines Instruments, insbesondere in starken Magnetfeldern, ohne dass diese dadurch (wesentlich) beeinflußt werden. Die Vorrichtung ist insbesondere bei bildgebenden Verfahren, wie der Computertomographie (CT), der Magnetresonanztomographie (MRT) oder dgl., einsetzbar. Die Erfindung betrifft außerdem insbesondere eine Vorrichtung zur Mammauntersuchung und/oder Mammabiopsie, die insbesondere bei der CT oder MRT einsetzbar ist.

Bisher bekannte Vorrichtungen für die Mammabiopsie in einem Magnetresonanztomographen sind nicht optimal gestaltet.

Die DE 295 21 366 U1 offenbart eine Vorrichtung für die Mammabiopsie in einem Magnetresonanztomographen. Eine zu untersuchende Mamma wird durch eine Platte mit einer Öffnung und einer zugeordneten Brustspule hindurch zwischen zwei beweglich aufgehängten Andruckplatten komprimiert. Die Andruckplatten sind entweder planar oder konkav ausgeführt. Weiter ist eine Positioniereinrichtung mit einer Aufnahme für eine Biopsieeinrichtung vorgesehen. Die Positioniereinrichtung ist manuell einstellbar. Die bekannte Vorrichtung gestattet keine optimale Untersuchung, insbesondere werden keine optimale Lagerung der zu untersuchenden Patientin und keine optimale Komprimierung der zu untersuchenden Mamma ermöglicht. Des Weiteren erfordert eine Positionierung der Aufnahme für die Biopsieeinrichtung oder dgl., dass die Vorrichtung jeweils aus einem Magnetresonanztomographen herausbewegt wird, wodurch die Untersuchung insgesamt wesentlich verlängert wird.

Die US 5,682,890 beschreibt die Verwendung eines thermoplastischen Netzes bei der Mammabiopsie. Das erwärmte thermoplastische Netz wird auf die zu untersuchende Mamma aufgebracht und ausgehärtet, um diese zu halten. Mittels einer manuell einstellbaren Positioniereinrichtung kann eine Biopsie unter Zuhilfenahme eines bildgebenden Verfahrens, wie dem CT, erfolgen. Die bekannte Vorrichtung gestattet keine optimale Untersuchung, insbesondere werden keine optimale Lagerung der zu untersuchenden Patientin und keine optimale Komprimierung der zu untersuchenden Mamma ermöglicht. Des Weiteren erfordert eine Positionierung der Aufnahme für die Biopsieeinrichtung oder dgl., dass die Vorrichtung jeweils aus einem Magnetresonanztomographen herausbewegt wird, wodurch die Untersuchung insgesamt wesentlich verlängert wird.

Die DE 10 2006 038 163 A1 offenbart eine Kompositionseinrichtung zur Mammauntersuchung, wobei eine Kompositionsplatte mit einer Mehrzahl von elastisch verformbaren Kompositionsplastenelementen gegen einen horizontalen Lagertisch zum Komprimieren einer zu untersuchenden Mamma bewegbar ist.

Die US 2007/0280412 A1 und US 2009/0213986 A1 beschreiben die Komprimierung einer Mamma mittels einer flexiblen Folie in Verbindung mit einem starren Andruckelement.

Die WO 2006/030406 A2 offenbart die Komprimierung einer Mamma mit einem flexiblen Bandmaterial gegen eine insbesondere konkave Anlagefläche.

Der vorliegenden Erfindung liegt die Aufgabe zugrunde, eine Vorrichtung zur Mammauntersuchung, insbesondere Biopsie, anzugeben, die eine optimierte Untersuchung bzw. Biopsie und/oder Positionierung, insbesondere in einem MRT, gestattet.

Die obige Aufgabe wird durch eine Vorrichtung gemäß Anspruch 1 gelöst. Vorteilhafte Weiterbildungen sind Gegenstand der Unteransprüche.

Gemäß vorliegenden Erfindung wird eine Vorrichtung zur Mammauntersuchung, insbesondere Mammabiopsie, mit einer Komprimierungseinrichtung zur Komprimierung einer zu untersuchenden Mamma vorgeschlagen. Unter dem Begriff "Komprimierung" ist generell auch ein Einklemmen oder sonstiges Festlegen zu verstehen. Insbesondere kann der Begriff "Komprimierungseinrichtung" auch im Sinne von "Halteeinrichtung zur Halterung einer zu untersuchenden Mamma" und der Begriff "Komprimierung" im Sinne von "Halterung" verstanden werden. Die Komprimierungseinrichtung kann also gemäß einer Ausführungsvariante die zu untersuchende Mamma auch lediglich halten oder in sonstiger Weise festlegen, ohne diese zu komprimieren bzw, ohne diese im Wesentlichen zu komprimieren.

Die Komprimierungseinrichtung weist ein erstes und ein zweites Element auf, wobei beide Elemente relativ zueinander verstellbar sind und zwischen beiden die Mamma aufnehmbar und komprimierbar ist. Ein Element bzw. das erste Element ist flexibel und/oder bandartig und/oder U-förmig und/oder transparent ausgebildet. Insbesondere ist das Element folienartig ausgebildet oder aus Folie hergestellt. Bedarfsweise kann die Folie auch auf der der Mamma zugewandten Seite klebbar ausgeführt sein. So kann eine optimale Untersuchung bzw. Komprimierung erreicht werden, da eine sehr gleichmäßige Druckverteilung und individuelle Anpassung ermöglicht wird. Außerdem gestattet die bevorzugte folienartige Ausbildung eine Biopsie durch das Element hindurch. Die bevorzugte transparente Ausbildung ist bei der Komprimierung und Untersuchung, insbesondere Biopsie, sehr vorteilhaft, da die von dem Element abgedeckte Mamma sichtbar bleibt.

Gemäß vorliegenden Erfindung bildet eines der beiden Elemente bzw. das zweite Element eine konvexe Anlagefläche für die Mamma und/oder ist vom ersten Element weggebogen oder weggeneigt. Überraschenderweise hat sich gezeigt, dass so eine bessere gleichmäßige Komprimierung der Mamma, insbesondere auch bei verschiedenen Patienten mit unterschiedlichen Mammas, ermöglicht wird. Dies ist auch einer besseren Untersuchung bzw. Biopsie zuträglich.

Vorzugsweise weist die Vorrichtung einen Liegeabschnitt zur Abstützung insbesondere eines Oberkörpers einer Frau bzw. Patientin, auf, wobei der Liegeabschnitt eine Öffnung für eine zu untersuchende Mamma aufweist. Der Liegeabschnitt weist einen quer zur Oberkörpererstreckung bzw. zur Längsstreckung der Vorrichtung und zu der Öffnung hin geneigten Hauptauflagebereich auf. Zur Untersuchung liegt die Frau bzw. Patientin vorzugsweise zumindest im wesentlichen horizontal, wobei die vorgenannte Querneigung dazu führt, dass die zu untersuchende Mamma möglichst tief liegt und der mittlere Bereich des Brustkorbs von der zu untersuchenden Mamma etwas nach oben weggeneigt ist. So wird eine Untersuchung der Mamma in einem Bereich benachbart zur Mitte des Brustkorbs und/oder in einem Bereich benachbart zur Achsel der zu untersuchenden Frau bzw. Patientin erleichtert.

Ein bevorzugter Aspekt der vorliegenden Erfindung liegt darin, dass der Zentrale bzw. mit einer seitlichen Öffnung für die Mamma versehene Liegeabschnitt zur Abstützung an entgegengesetzten Enden jeweils wahlweise mit einer Kopfabstützung und einem zusätzlichen Liegeabschnitt verbindbar ist. So kann eine Liege bzw. Abstützungseinrichtung gebildet werden, die ein Drehen des zentralen Liegeabschnitts gestattet, so dass die Vorrichtung wahlweise zur Untersuchung der rechten oder der linken Mamma eingesetzt bzw. umgebaut werden kann. Außerdem wird ein insbesondere modulartiger Aufbau einer Liege bzw. Abstützung für die zu untersuchende Frau bzw. Patientin ermöglichst, wobei eine entsprechende Anpassung an die jeweiligen Erfordernisse sehr leicht erfolgen kann.

Ein bevorzugter Aspekt der vorliegenden Erfindung betrifft eine Positioniereinrichtung mit einer Aufnahme für einen zu positionierenden Gegenstand, wie einer Biopsienadel oder sonstigen Biopsieeinrichtung. Die Gestaltung dieser Aufnahme ermöglicht vorzugsweise die Aufnahme, Führung und Fixierung hinsichtlich des Durchmessers verschieden aufgebauter Biopsiesysteme. Die Aufnahme ist vorzugsweise als Einwegbauteil austauschbar gestaltet, da sie in direktem Kontakt mit Biopsiesystem und Körperflüssigkeiten steht. Sie ist vorzugsweise auch oberhalb der für die Mamma vorgesehenen Öffnung positionierbar. Hierdurch lassen sich Untersuchungen bzw. Biopsien auch seitlich an einer Mamma bzw. benachbart zu der Achsel bzw. an der Seite der zu untersuchenden Frau bzw. Patientin durchführen.

Ein bevorzugter Aspekt der vorliegenden Erfindung ist darauf gerichtet, dass die Positioniereinrichtung hydraulisch steuerbar und/oder fernsteuerbar ist. Dies gestattet eine wesentlich vereinfachte Untersuchung, da die Positioniereinrichtung bzw. die Vorrichtung zur Untersuchung mit der Positioniereinrichtung auch in sehr beengten räumlichen Verhältnissen, insbesondere auch direkt in einem Magnetresonanztomographen oder dgl., zur Positionierung eingesetzt werden kann bzw. können, insbesondere ohne dass ein Herausbewegen aus dem Magnetresonanztomographen für die Änderung der Position der Aufnahme erforderlich ist. Dementsprechend können Untersuchungen und insbesondere Biopsien wesentlich schneller - besonders bevorzugt auch innerhalb heute üblicher, nur sehr kurzer Narkosen - durchgeführt werden.

Ein bevorzugter Aspekt der vorliegenden Erfindung ist darauf gerichtet, dass die Positioniereinrichtung eine Umlenkeinrichtung zur Umwandlung einer Linearbewegung eines Antriebs in einer quer dazu verlaufenden Bewegung oder Verstellung der Aufnahme aufweist. Insbesondere weist die Positioniereinrichtung zwei Antriebe mit zumindest im Wesentlichen parallelen Linearbewegungen auf, durch die die Aufnahme zweidimensional positionierbar ist. Die Antriebe liegen vorzugsweise unmittelbar übereinander und/oder nebeneinander. So werden ein sehr kompakter Aufbau und insbesondere auch eine Intergation in eine Patientenliege oder dgl. ermöglicht.

Weitere Vorteile, Merkmale, Eigenschaften und Aspekte der vorliegenden Erfindung ergeben sich aus der nachfolgenden Beschreibung bevorzugter Ausführungsformen anhand der Zeichnung. Es zeigt:
- Fig. 1: eine perspektivische Ansicht einer vorschlagsgemäßen, Vorrichtung gemäß einer ersten Ausführungsform mit einem zentralen Liegeabschnitt, einer Komprimierungseinrichtung und einer Positioniereinrichtung;
- Fig. 2: einen schematischen Schnitt der Vorrichtung bzw. Komprimierungseinrichtung;
- Fig. 3: eine Draufsicht der Komprimierungseinrichtung;
- Fig. 4: eine Seitenansicht der Positioniereinrichtung;
- Fig. 5: eine Seitenansicht des Liegeabschnitts mit der Positioniereinrichtung;
- Fig. 6: eine perspektivische Ansicht einer vorschlagsgemäßen Vorrichtung gemäß einer zweiten Ausführungsform mit einem zentralen Liegeabschnitt, einer Komprimierungseinrichtung und einer Positioniereinrichtung; und
- Fig. 7: eine Draufsicht der Komprimierungseinrichtung gemäß der zweiten Ausführungsform.

In den Figuren werden für gleiche oder ähnliche Bauteile und Komponenten die gleichen Bezugszeichen verwendet, wobei sich entsprechende oder ähnliche Vorteile und Aspekte bzw. Eigenschaften ergeben, auch wenn eine wiederholte Beschreibung weggelassen ist.

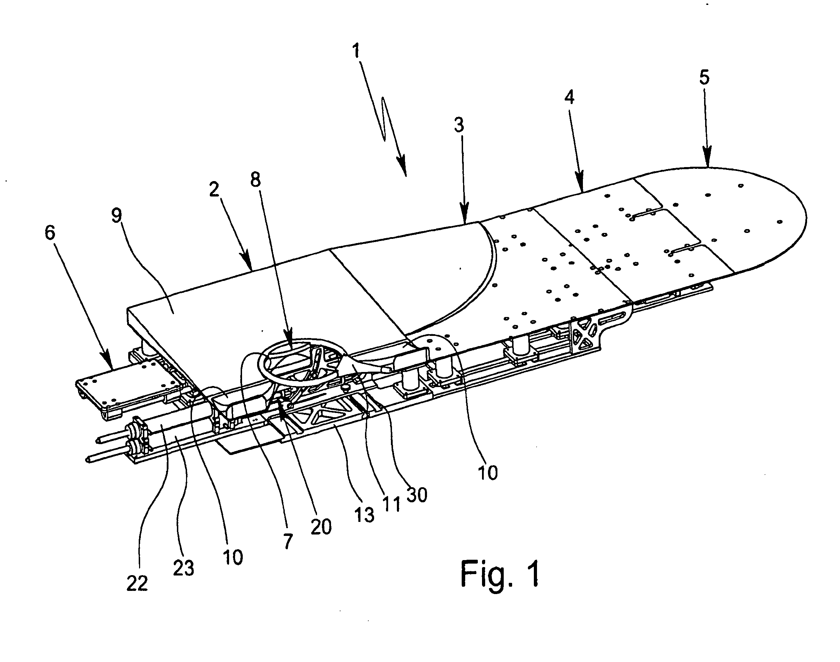

Fig. 1 zeigt in einer perspektivischen Darstellung eine vorschlagsgemäße Vorrichtung 1 gemäß einer ersten bevorzugten Ausführungsform. Die Vorrichtung dient vorzugsweise einer Untersuchung, insbesondere Biopsie, einer nicht dargestellten Person, wie einer Frau oder Patientin, besonders bevorzugt einer zu untersuchenden Mamma (Brust) einer Frau bzw. Patientin. Die Vorrichtung 1 dient alternativ oder zusätzlich einer Komprimierung der zu untersuchenden Mamma. Die Vorrichtung 1 dient alternativ oder zusätzlich der Positionierung einer Aufnahme für einen zu positionierenden Gegenstand, wie einer Biopsieeinrichtung oder dgl. Die Vorrichtung 1 ist insbesondere zum Einsatz in einem bildgebenden Verfahren, insbesondere in der CT oder MRT bzw. in einem Computertomographen oder Magnetresonanztomographen (nicht dargestellt), vorgesehen bzw. ausgebildet. Nachfolgend wird ein bevorzugter Aufbau der Vorrichtung 1, insbesondere zur Realisierung der vorgenannten Zwecke und Aspekte, erläutert.

Die Vorrichtung 1 weist einen (zentralen) Liegeabschnitt 2 sowie optional einen zusätzlichen Liegeabschnitt 3, insbesondere mehrere zusätzliche Liegeabschnitte 3, 4 und 5, und/oder eine Kopfabstützung 6 auf. Vorzugsweise wird eine Liege bzw. Liegefläche zur vorzugsweise horizontalen Lagerung oder Abstützung einer nicht dargestellten Person, insbesondere einer Frau bzw. Patientin, von der Vorrichtung 1 bzw. von den Liegeabschnitten 2, 3, 4, 5 und der optionalen Kopfabstützung 6 gebildet. Jedoch sind auch andere konstruktive Lösungen möglich.

Die Vorrichtung 1 bzw. der Liegeabschnitt 2 weist vorzugsweise eine Durchbrechung oder Öffnung 7 auf, durch die sich eine zu untersuchende Mamma (in Fig. 1 nicht dargestellt) nach unten in eine in Fig. 1 nur angedeutete Komprimierungseinrichtung 8 der Vorrichtung 1 erstrecken kann. Die Vorrichtung 1 bzw. die davon gebildete Liege oder Liegefläche erstreckt sich zumindest im Wesentlichen horizontal. Die Haupterstreckungsrichtung der zu untersuchenden Mamma (vom Brustansatz zur Brustspitze) erstreckt sich vorzugsweise zumindest im Wesentlichen vertikal bzw. von oben nach unten beim vorliegenden bevorzugten Ausführungsbeispiel.

Die Öffnung 7 ist vorzugsweise auf einer Seite, insbesondere benachbart zu oder an einem Längsrand oder einer Längsseite, der Vorrichtung 1 bzw. des Liegeabschnitts 2 bzw. bezüglich einer Oberkörperlängsachse einer nicht dargestellten, zu untersuchenden Frau bzw. Patientin angeordnet. Bei der Darstellung gemäß Fig. 1 ist die Öffnung 7 auf der linken Seite angeordnet und die Vorrichtung 1 für die Untersuchung einer linken Mamma der zu untersuchenden Frau bzw. Patientin vorgesehen.

Die Vorrichtung 1 ist vorzugsweise derart ausgebildet, dass die Öffnung 7 wahlweise auf der rechten oder linken Seite angeordnet werden kann bzw. wahlweise die rechte oder linke Mamma untersucht werden kann. Hierzu ist der Liegeabschnitt 2 vorzugsweise an seinen entgegengesetzten Enden jeweils wahlweise mit der optionalen Kopfabstützung 6 einerseits oder dem nächsten zusätzlichen Liegeabschnitt 3 oder einem sonstigen Liegeabschnitt 3 andererseits verbindbar. Insbesondere kann der Liegeabschnitt 2 in der Darstellung gemäß Fig. 1 gedreht werden, so dass die Öffnung 7 nicht auf der linken, sondern bei Bedarf auf der rechten Seite zu liegen kommt.

Besonders bevorzugt sind die Liegeabschnitte 2 bis 5 und die Kopfabstützung 6 steckbar und/oder werkzeuglos miteinander verbindbar. Dies gestattet einen sehr einfachen Umbau der Vorrichtung 1 bzw. einen sehr einfachen Wechsel der Öffnungsseite.

Bedarfsweise können auch unterschiedlich geformte oder unterschiedlich große Liegeabschnitte 2 bis 5 - insbesondere je nach Zweck der Untersuchung und/oder Größe des zu untersuchenden Menschen bzw. der zu untersuchenden Frau oder Patientin - verwendet bzw. miteinander kombiniert oder zusammengebaut werden.

Beim Darstellungsbeispiel ist die Kopfabstützung 6 vorzugsweise mittels einer entsprechenden Abstützeinrichtung teleskopisch bzw. schlittenartig vorzugsweise in Körperlängserstreckung bzw. Längserstreckung der Vorrichtung 1 verstellbar. Jedoch sind auch andere konstruktive Lösungen möglich.

Der Zentrale bzw. mit der Öffnung 7 versehene Liegeabschnitt 2 dient vorzugsweise der Auflagerung oder Abstützung eines (nicht dargestellten) Oberkörpers einer zu untersuchenden Frau bzw. Patientin.

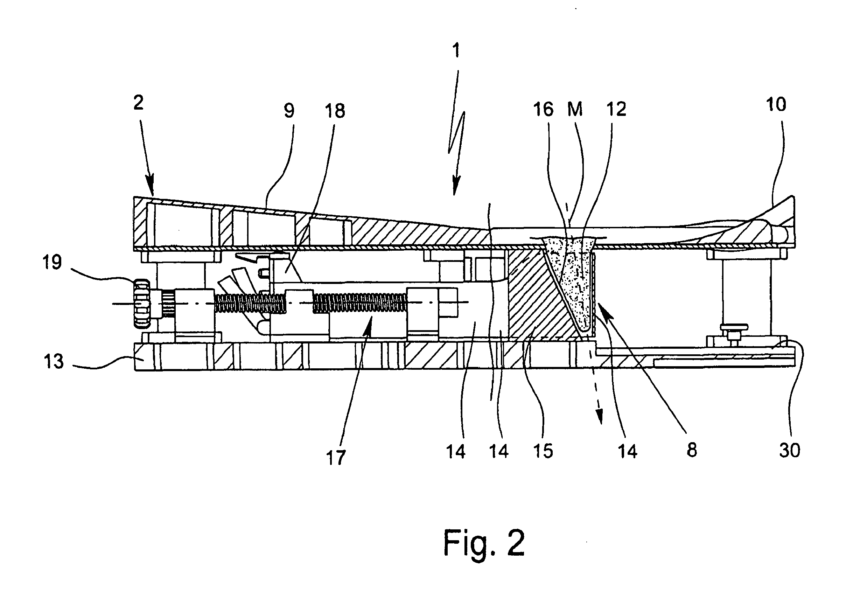

Der Liegeabschnitt 2 weist vorzugsweise einen quer zur Oberkörperlängserstreckung bzw. zu der Längserstreckung der Vorrichtung 1 geneigten Hauptauflagebereich 9 auf, der in Fig. 1 angedeutet und in der Schnittdarstellung gemäß Fig. 2 besser zu erkennen ist. Der Hauptauflagebereich 9 ist vorzugsweise zur Öffnung 7 hin geneigt. Der Hauptauflagebereich 9 ist in der zumindest im Wesentlichen horizontalen Gebrauchslage der Vorrichtung 1 zur Horizontalen geneigt. Der Hauptauflagebereich 9 ist insbesondere derart geneigt dass die zu untersuchende, durch die Öffnung 7 hindurchtretende Mamma 12 - die in Fig. 2 angedeutet ist - möglichst tief liegt und/oder möglichst frei von allen Seiten zugänglich ist.

Die Vorrichtung 1 bzw. der Liegeabschnitt 2 weist vorzugsweise mindestens einen Seitenauflagebereich 10 auf, der ebenfalls quer zur Oberkörperlängserstreckung bzw. zur Längserstreckung der Vorrichtung 1 und zur Horizontalen geneigt ist, jedoch entgegengesetzt zum Hauptauflagebereich 9. Insbesondere bildet der Seitenauflagebereich 10 ein seitliches Widerlager für die Frau bzw. Patientin, um ein seitliches Herunterrutschen von dem Hauptauflagebereich 9 zu verhindern bzw. zu begrenzen.

Der Seitenauflagebereich 10 schließt sich insbesondere an eine Längsseite des Hauptauflagebereichs 9 an, insbesondere derart, dass zwischen dem Hauptauflagebereich 9 und dem Seitenauflagebereich 10 ein angepaßter oder abgerundeter Übergang entsteht und/oder ein Tal gebildet wird, wie insbesondere in Fig. 2 angedeutet. In diesem Übergangsbereich bzw. Tal oder einer Verlängerung in Längsrichtung davon ist dann vorzugsweise die Öffnung 7 angeordnet.

Die Öffnung 7 ist vorzugsweise randseitig offen ausgebildet. Dementsprechend ist der Seitenauflagebereich 10 vorzugsweise im Bereich der Öffnung 7 ausgespart. Beim Darstellungsbeispiel schließt sich rechts und links von der Öffnung 7 jeweils an den Hauptauflagebereich 9 bzw. dessen Längskante oder Längsseite ein Seitenauflagebereich 10 an, wie in Fig. 1 angedeutet. Jedoch sind auch andere konstruktive Lösungen möglich.

Um die Lagerung des Patienten bzw. der Patientin zu optimieren, kann die Querneigung des Hauptauflagebereichs 9 und/oder der Seitenauflagebereiche 10 bedarfsweise auch einstellbar oder durch auflegbare Polster oder dergleichen variierbar sein.

Die Vorrichtung 1 ist vorzugsweise zur Verwendung bzw. zum Einsatz bei einem bildgebenden Verfahren bzw. in einem Magnetresonanztomographen (Kernspinntomographen) oder dgl. vorgesehen. Insbesondere ist dann eine Brustspule 11 im Bereich der Öffnung 7 bzw. um die Öffnung 7 herum anordenbar, wie in Fig. 1 angedeutet.

Die Vorrichtung 1 ist vorzugsweise zumindest im Wesentlichen aus Kunststoff und/oder aus einem sonstigen MRT-verträglichen Material hergestellt.

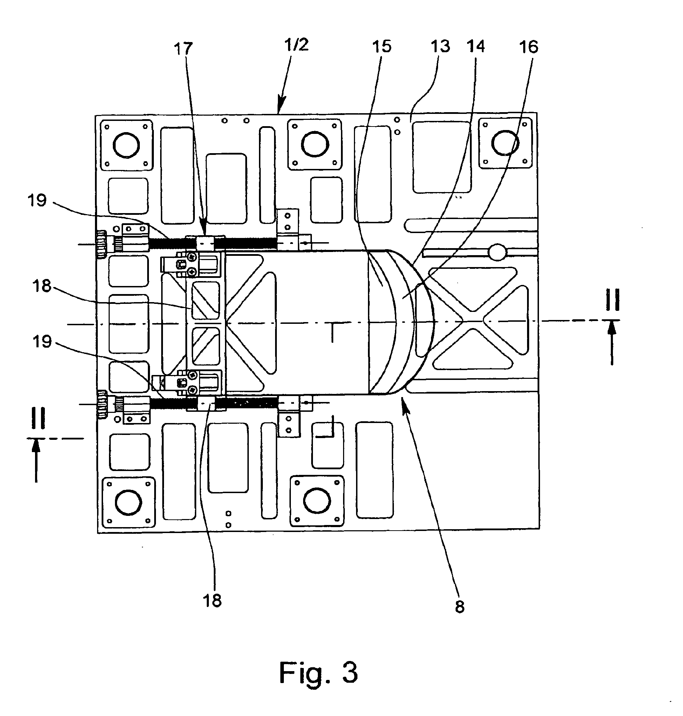

Nachfolgend wird ein bevorzugter Aufbau der Komprimierungseinrichtung 8 insbesondere anhand des Vertikalschnitts gemäß Fig. 2 und anhand des Horizontalschnitts gemäß Fig. 3 erläutert. Wie aus Fig. 3 ersichtlich, verläuft der Schnitt gemäß Fig. 2 im Wesentlichen wie durch Linie II-II in Fig. 3 angedeutet.

Die Komprimierungseinrichtung 8 dient der Komprimierung bzw. Festlegung bzw. dem Halten oder Einklemmen der zu untersuchenden Mamma 12, die in Fig. 2 nur schematisch angedeutet ist.

Die Komprimierungseinrichtung 8 ist vorzugsweise unterhalb der Öffnung 7 und/oder zwischen dem Hauptauflagebereich 9 und einer Bodenplatte 13 des Liegeabschnitts 2 angeordnet. Jedoch sind auch andere konstruktive Lösungen möglich.

Die Komprimierungseinrichtung 8 weist ein erstes Element 14 und ein zweites Element 15 auf, wobei die beiden Elemente 14 und 15 relativ zueinander verstellbar sind und zwischen beiden die Mamma 12 aufnehmbar und komprimierbar ist.

Das erste Element 14 ist vorzugsweise flexibel, bandartig und/oder folienartig ausgebildet oder aus Folie hergestellt.

Das erste Element 14 ist vorzugsweise aus einer für Operationen einsetzbaren Folie hergestellt. Jedoch kann grundsätzlich auch ein Netz oder dgl. oder ein sonstiges flexibles Material, das insbesondere ausreichend zugfest ist, eingesetzt werden.

Das erste Element 14 ist vorzugsweise zumindest im Wesentlichen nicht dehnbar ausgebildet. Jedoch kann das erste Element 14 bzw. die Folie auch dehnbar ausgeführt sein.

Gemäß einer Ausführungsvariante ist das erste Element 14 bzw. die Folie klebend mit auf der Mamma 12 verbindbar bzw. mit einer Klebeschicht versehen.

Das erste Element 14 bzw. die Folie ist vorzugsweise zumindest partiell transparent ausgebildet. Dies ist bei einer Untersuchung zuträglich, da so die darunter liegende Mamma 12 insbesondere für einen Arzt sichtbar bleibt.

Das erste Element 14 ist vorzugsweise derart ausgebildet, dass es von einer Untersuchungseinrichtung, insbesondere einer Biopsienadel oder sonstigen Biopsieeinrichtung (nicht dargestellt), durchdringbar bzw. penetrierbar oder durchstechbar ist. Insbesondere gestattet das erste Element 14 also eine Biopsie an einer beliebigen Stelle. Dies ermöglicht eine optimale Untersuchung.

Der schematischen Darstellung gemäß Fig. 2 ist zu entnehmen, dass das erste Element 14 in seiner Längserstreckung zumindest im Wesentlichen in einer Horizontalebene verläuft und in seiner Quererstreckung zumindest im wesentlichen vertikal ausgerichtet ist, insbesondere auch bei komprimierter Mamma 12.

Besonders bevorzugt verläuft das erste Element 14 zumindest im Wesentlichen U-förmig und/oder um das zweite Element 15 herum, wie insbesondere in Fig. 3 angedeutet.

Das zweite Element 15 ist vorzugsweise massiv bzw. starr oder zumindest im Wesentlichen nicht verformbar ausgebildet. Es kann jedoch auch zumindest etwas elastisch verformbar sein.

Das zweite Element 15 bildet eine Wiederlager- bzw. Anlagefläche 16 für die Mamma 12. Die Mamma 12 ist also vorzugsweise zwischen der Anlagefläche 16 und dem ersten Element 14 bzw. der Folie komprimierbar oder einklemmbar.

Aus Fig. 2 ist ersichtlich, dass die Anlagefläche 16 vorzugsweise vom ersten Element 14 weggebogen oder weggeneigt ist. Dies gilt insbesondere in einem in der Gebrauchslage vertikalen Schnitt in dem Bereich, in dem die zu untersuchende Mamma 12 zur Anlage kommt. Die Neigung verläuft dabei so, dass zu der (in Fig. 2 unten liegenden) Brustspitze hin der Abstand zwischen der Anlagefläche 16 und dem unmittelbar benachbarten Bereich des ersten Elements 14 abnimmt.

Die Anlagefläche 16 ist vorzugsweise konvex ausgebildet, insbesondere in einer Ebene parallel und/oder in einer Ebene senkrecht zur Mammaerstreckungsrichtung (vom Brustansatz zur Brustspitze, also in der Darstellung gemäß Fig. 2 von oben nach unten gemäß Pfeil M bzw. im Wesentlichen vertikal oder quer bzw. senkrecht zur Horizontalen oder Haupterstreckungsebene der Vorrichtung 1). Anstatt der bevorzugten gewölbten Ausführung der Anlagefläche 16 kann diese im Vertikalschnitt auch zumindest im Wesentlichen geradlinig verlaufen bzw. gleichmäßig geneigt, also nicht gewölbt sein.

Das erste Element 14 ist vorzugsweise mittels einer Spanneinrichtung 17 der Komprimierungseinrichtung 8 relativ zum zweiten Element 15 bzw. zur Anlagefläche 16 verstellbar und insbesondere spannbar.

Die Spanneinrichtung 17 greift vorzugsweise an einem Ende oder an beiden Enden des ersten Elements 14 an. Insbesondere ist das erste Element 14 bzw. die Folie in ein entsprechendes bzw. geeignetes Spannelement 18 einhängbar, einklemmbar und/oder in sonstiger Weise damit verbindbar. Das Spannelement 18 ist vorzugsweise schlittenartig geführt und/oder vorzugsweise mittels mindestens einer Spannschraube 19, beim Darstellungsbeispiel zweiter Spannschrauben 19, verstellbar. Jedoch sind auch andere konstruktive Lösungen möglich.

Die Spanneinrichtung 17 ist beim Darstellungsbeispiel vorzugsweise manuell betätigbar, insbesondere durch Drehen der Spannschraube(n) 19 oder dgl.

Das erste Element 14 ist vorzugsweise lösbar mit der Spanneinrichtung 17 bzw. dem Spannelement 18 verbindbar, und insbesondere generell auswechselbar, so dass für jede Untersuchung ein neues erstes Element 14 einsetzbar ist. Entsprechend kann das zweite Element 15 auch auswechselbar und/oder zum Reinigen und Desinfizieren bzw. Sterilisieren demontierbar ausgebildet sein.

Die dargestellte Komprimierungseinrichtung 8 stellt lediglich ein besonders bevorzugtes Ausführungsbeispiel dar. Andere konstruktive Ausgestaltungen sind jedoch ebenso möglich.

Die vorschlagsgemäße Komprimierungseinrichtung 8 und insbesondere der Einsatz des flexiblen bzw. folienartigen ersten Elements 14 gestatten eine besonders gleichmäßige und damit definiertere Komprimierung und Festlegung der Mamma 12. Die Gestaltung der Komprimierungseinrichtung 8 ermöglicht nicht nur eine definierte Komprimierung der Mamma 12, sondern komprimiert das (viskose) Gewebe der Mamma 12 vorzugsweise derart gleichmäßig und wunschgemäß, dass vorhandene Fremdkörper und Verhärtungen (wie Karzinome etc.) in der aktuellen Position für die Biopsie und während der Biopsie fixiert werden. Das unerwünschte Ausweichen eines Fremdkörpers während der eigentlichen Gewebeentnahme wird dadurch erheblich erschwert bzw. verhindert.

Es wird also auch ein Verfahren zum Komprimieren einer Mamma 12 vorgeschlagen, wobei die Mamma 12 mittels einer Folie oder eines sonstigen flexiblen Elements 14 komprimiert wird, insbesondere gegen ein feststehendes zweites Element 15 gespannt bzw. dazwischen eingeklemmt wird.

Die Vorrichtung 1 weist weiter vorzugsweise eine Positioniereinrichtung 20 auf. Die Positioniereinrichtung 20 ist in Fig. 1 nur schematisch angedeutet. Sie ist insbesondere unterhalb der Öffnung 7 und/oder zwischen dem Hauptauflagebereich 9 und/oder einem Seitenauflagebereich 10 und der Bodenplatte 13 des Liegeabschnitts 2 andererseits angeordnet.

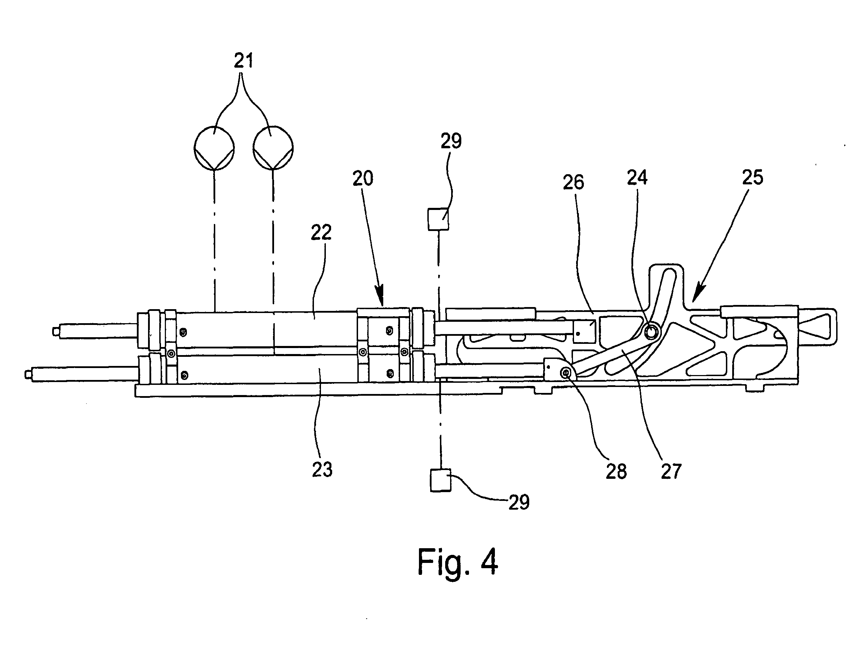

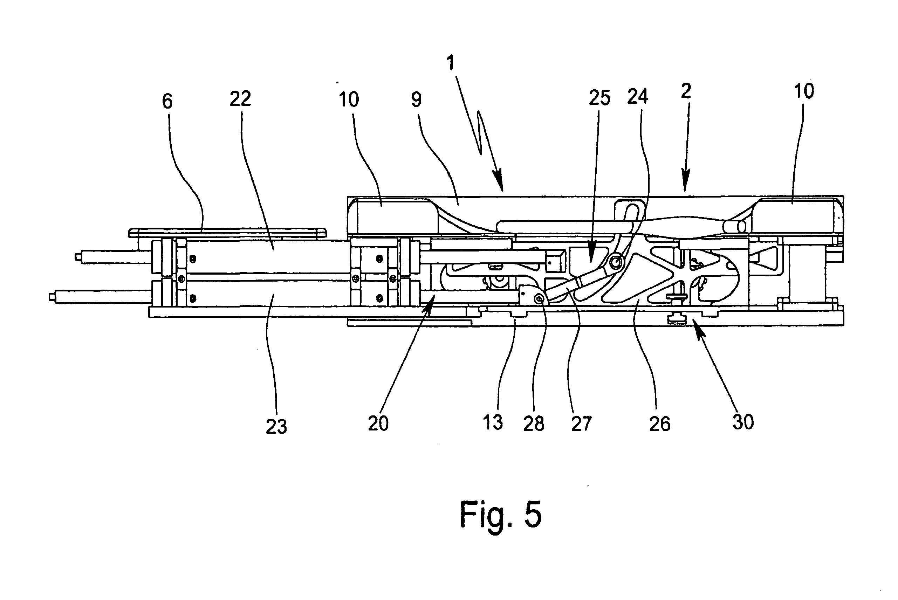

Die Positioniereinrichtung 20 stellt vorzugsweise eine Baueinheit dar, die lösbar mit der Vorrichtung 1 verbindbar ist. Die Positioniereinrichtung 20 ist in den Fig. 2 und 3 nicht dargestellt. Fig. 4 zeigt die ausgebaute Positioniereinrichtung 20 mit zwei zugeordneten Dosierpumpen 21. Fig. 5 zeigt die Positioniereinrichtung 20 in einer Seitenansicht im in die Vorrichtung 1 eingebauten Zustand, wobei hier nur der Liegeabschnitt 2 und keine zusätzlichen Liegeabschnitte 3 bis 5 dargestellt sind.

Die Positioniereinrichtung 20 weist vorzugsweise einen Antrieb bzw. eine Antriebseinheit auf. Der Antrieb bzw. die Antriebseinheit arbeitet insbesondere hydraulisch bzw. ist hydraulisch antreibbar. Besonders bevorzugt handelt es sich um einen Stellantrieb.

Beim Darstellungsbeispiel weist die Positioniereinrichtung 20 zwei Antriebseinheiten bzw. Antriebe, besonders bevorzugt in Form von Hydraulikzylindern 22 und 23, auf. Die Positioniereinrichtung 20 und deren Antriebe sind also vorzugsweise nicht elektrisch, sondern mechanisch oder insbesondere hydraulisch steuerbar und/oder fernsteuerbar.

Besonders bevorzugt wird Wasser als Hydraulikflüssigkeit zur Steuerung eingesetzt. Versuche haben gezeigt, dass dies überraschenderweise problemlos in einen Magnetresonanztomographen möglich ist.

Die Positioniereinrichtung 20 weist eine vorzugsweise hülsenartige oder hohle Aufnahme 24 für einen zu positionierenden oder insbesondere längsverschieblich zu haltenden und/oder vorzugsweise fixierenden Gegenstand, wie eine Biopsieeinrichtung, Biopsienadel oder dgl. (nicht dargestellt), auf. Die Positioniereinrichtung 20 gestattet insbesondere eine zweidimensionale Positionierung, insbesondere also eine zweidimensionale Bewegung oder Verstellung der Aufnahme 24 bzw. eine Verstellung oder Bewegung der Aufnahme 24 in einer Ebene, beim Darstellungsbeispiel bei der Gebrauchslage der Vorrichtung 1 vorzugsweise zumindest im wesentlichen in einer Vertikalebene.

Beim Darstellungsbeispiel erzeugen die Antriebe Linearbewegungen. Insbesondere verlaufen die beiden Linearbewegungen der beiden Antriebe der Positioniereinrichtung 20 vorzugsweise zumindest im Wesentlichen parallel zueinander. Die Hydraulikzylinder 22 und 23 sind vorzugsweise parallel zueinander ausgerichtet und/oder vorzugsweise unmittelbar nebeneinander und/oder übereinander angeordnet. Dies gestattet einen besonders kompakten Aufbau.

Die Positioniereinrichtung 20 weist vorzugsweise eine Umlenkeinrichtung 25 zur Umwandlung mindestens einer Linearbewegung eines Antriebs in eine quer dazu verlaufende Bewegung oder Verstellung der Aufnahme 24 auf. Besonders bevorzugt weist die Umlenkeinrichtung 25 eine insbesondere linear verschiebbare Kulisse 26 und einen von oder and er Kulisse 26 geführten, separat verstellbaren Positionsarm 27 auf, der der Aufnahme 24 zugeordnet ist bzw. diese trägt, hält oder bildet.

Die Kulisse 26 und der Positionsarm 27 sind vorzugsweise jeweils von einem zugeordneten Antrieb separat antreibbar bzw. bewegbar. Insbesondere ist die Kulisse 26 linear verschiebbar, hier mittels des Hydraulikzylinders 22.

Der Positionsarm 27 ist vorzugsweise über ein Gelenk 28 mit seinem zugeordneten Antrieb, hier dem Hydraulikzylinder 23, verbunden. Insbesondere ist das Gelenk 28 linear hin- und her bewegbar, wobei der Positionsarm 27 im Bereich seines anderen Endes bzw. der Aufnahme 24 an oder von der Kulisse 26, insbesondere in einem Langloch, das hier vorzugsweise gebogen ist, geführt wird. So wird die Linearbewegung des dem Positionsarm 27 zugeordneten Antriebs bzw. Hydraulikzylinders 23 in eine quer dazu verlaufende Bewegung bzw. Verstellung umgewandelt.

Durch die überlagerte Bewegung der Kulisse 26 und des Positionsarms 27 wird eine zweidimensionale Positionierung ermöglich, und zwar bei der Darstellungsgemäß Fig. 4 in der Zeichenebene. Jedoch sind auch andere konstruktive Lösungen möglich.

Die Antriebe bzw. Hydraulikzylinder 22 und 23 werden vorzugsweise von zugeordneten Dosierpumpen 21 angetrieben. Besonders bevorzugt erfolgt lediglich eine Steuerung oder Ansteuerung der Antriebe bzw. Hydraulikzylinder 22, 23. Ausgehend von einer Anfangs- oder Ausgangslage wird über die Dosierpumpen 21 jeweils eine in Abhängigkeit von der gewünschten Endlage oder Positionierung vorbestimmte Menge an Hydraulikflüssigkeit zu geführt. Versuche haben gezeigt, dass so eine für den Einsatz in der Medizin ausreichend genaue Positionierung sehr leicht erreichbar ist.

Die Antriebe bzw. Hydraulikzylinder 22 und 23 können bedarfsweise gegen nicht dargestellte Rückstellelemente, wie Rückstellfedern oder dergleichen, arbeiten. Insbesondere kann dann nach Ablassen der Hydraulikflüssigkeit ein automatisches Rückstellen in die Ausgangslage erfolgen.

Die Antriebe bzw. Hydraulikzylinder 22 und 23 können entweder jeweils einfach wirkend - also nur in eine Richtung wirkend - oder doppelt wirkend - also in beiden Richtungen wirkend - ausgebildet sein und entsprechend hydraulisch - ggf. über entsprechende Ventile oder mehrere Dosierpumpen 21 - angesteuert werden.

Die Dosierpumpen 21 sind vorzugsweise über geeignete (lange) Leitungen angeschlossen und können dementsprechend auch weit entfernt von der Positionseinrichtung 20, insbesondere weit außerhalb eines Magnetresonanztomographen, aufgestellt und betrieben werden.

Die Dosierpumpen 21 sind der Positionierteneinrichtung 20 bzw. den Antrieben bzw. Hydraulikzylindern 22 und 23 zugeordnet. Die Positioniereinrichtung 20 kann die Dosierpumpen 21 jedoch auch umfassen.

Eine elektronische Steuereinrichtung für die Ansteuerung der Dosierpumpen 21 bzw. Antriebe mit dem notwendigen Ansteuerungsprogramm ist vorzugsweise vorgesehen, jedoch nicht dargestellt.

Die elektronische Steuereinheit zeichnet sich insbesondere dadurch aus, dass sie gewünschte Koordinaten, beispielsweise von einem Magnetresonanztomographen bestimmte Koordinaten, für die Positionierung in entsprechende Zylinderpositionen bzw. Positionen der Antriebe umrechnet und aus der Ausgangposition und/oder gemessenen Position die erforderliche Hydraulikmenge bzw. erforderliche Anzahl von Steuerimpulsen für die Ansteuerung der Schrittmotoren berechnet und dann diese Steuerimpulse erzeugt und ausgibt. Jedoch sind auch andere Arten der Steuerung bzw. Ansteuerung möglich.

Den Antrieben bzw. der Kulisse 26 und dem Positionsarm 27 können auch Positionssensoren, Endlagenschalter, optische oder sonstige Sensoren 29 oder dergleichen zugeordnet sein, wie in Fig. 4 angedeutet. Diese können zur Überprüfung oder Erkennung der Rückstellung der Antriebe oder auch beispielsweise zur Erfassung der aktuellen Position der Antriebe oder ggf. auch zur Regelung der Antriebe bzw. der Positionierung eingesetzt werden.

Die schematische Seitenansicht gem. Fig. 5 zeigt, dass die Positioniereinrichtung 20 vorzugsweise derart ausgebildet ist, dass die Aufnahme 24 auch oberhalb der Öffnung 7 positionierbar ist. In Fig. 5 ist erkennbar, dass sich hierzu die Kulisse 26 mit ihrer Führung für den Positionierarm 27 von unten (die Positioniereinrichtung 20 ist unterhalb der Öffnung 7 angeordnet) durch die Öffnung 7 hindurch über die Öffnung 7 hinaus nach oben erstreckt. Dies gestattet auch eine sehr hohe seitliche Positionierung der Aufnahme 24 insbesondere an der Längsseite einer Patientin bzw. im Bereich der Achsel einer Patientin, wo eine Biopsie normalerweise schwierig oder gar nicht möglich ist.

Die vorschlagsgemäße Positioniereinrichtung 20 ist hinsichtlich der Bauhöhe kompakt ausgeführt und kann so problemlos unter einer Auflagefläche angeordnet oder in eine Liege bzw. die Vorrichtung 1 integriert werden.

Die Positioniereinrichtung 20 ist vorzugsweise auch quer zur zweidimensionalen Verstellbarkeit der Aufnahme 24 verstellbar oder justierbar, beispielsweise über eine entsprechende Schlittenführung 30.

Vorzugsweise ist es möglich, die Komprimierungseinrichtung 8 und/oder die Positioniereinrichtung 20 insbesondere um 90° Grad in der Horizontalebene gedreht unterhalb der Öffnung 7 anzuordnen, um die zu untersuchende Mamma 12 auch an oder von anderen Seiten untersuchen bzw. biopsieren zu können. Die Vorrichtung 1 ist vorzugsweise entsprechend ausgebildet, dass die Komprimierungseinrichtung 8 und/oder die Positioniereinrichtung 20 in verschiedenen Lagen einbaubar ist bzw. sind. Die Positioniereinrichtung 20 ist vorzugsweise derart ausgebildet bzw. an die Vorrichtung 1 angepaßt, dass die Positioniereinrichtung 20 auch quer im Liegeabschnitt 2 angeordnet werden kann, ohne seitlich über diesen hinaus zu stehen, insbesondere wahlweise auf der dem zusätzlichen Liegeabschnitt 3 zugewandeten Seite der Mamma 12 oder auf der der Kopfabstützung 6 zugewandten Seite der Mamma 12.

Die Positioniereinrichtung 20 kann ebenso wie die sonstige Vorrichtung 1 zumindest im Wesentlichen aus Kunststoff, insbesondere aus POM und/oder Teflon, hergestellt werden. Jedoch können für die Antriebe, insbesondere deren Zylinder, Kolben oder Kolbenstangen auch sonstige Werkstoffe, wie Metall, eingesetzt werden.

Es ist anzumerken, dass die Positioniereinrichtung 20 auch generell für die Positionierung insbesondere von medizinischen Instrumenten oder dergleichen, besonders bevorzug in starken Magnetfeldern, eingesetzt werden kann. Beim Einsatz bei der MRT bzw. in einem Magnetresonanztomographen ist vorteilhaft, dass die MRT von der Positioniereinrichtung 20 ebenso wenig wie von der Vorrichtung 1 ansonsten nicht oder nicht merklich beeinflußt wird.

Nachfolgend wird eine zweite Ausführungsform der vorschlagsgemäßen Vorrichtung 1 anhand der weiteren Figuren näher erläutert. Hierbei werden nur wesentliche Unterschiede gegenüber der ersten Ausführungsform beschrieben. Die bisherigen Ausführungen und Erläuterungen gelten insbesondere entsprechend oder ergänzend, auch wenn eine wiederholte Beschreibung weggelassen ist.

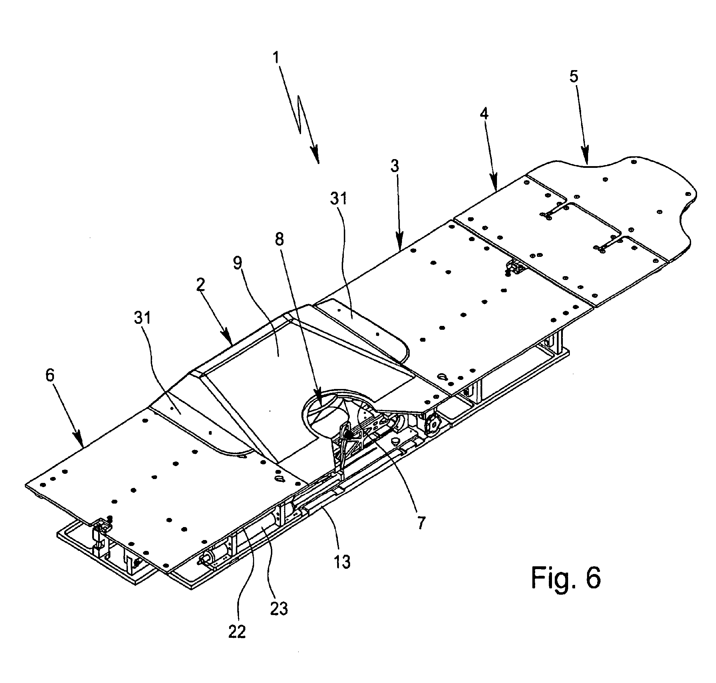

Fig. 6 zeigt in einer perspektivischen Ansicht eine zweite Ausführungsform der vorschlagsgemäßen Vorrichtung 1. Bei der zweiten Ausführungsform ist der Kopfabschnitt 6 vorzugsweise als ein breiterer Liegeabschnitt, insbesondere zur Abstützung oder Auflage der Arme seitlich neben dem Kopf einer zu untersuchenden Frau bzw. Patientin (nicht dargestellt) ausgebildet. Besonders bevorzugt ist der Kopfabschnitt 6 genauso breit wie der zentrale Liegeabschnitt 2 ausgebildet.

Vorzugsweise ist der Kopfabschnitt 6 genauso wie der sich auf der anderen Seite an den zentralen Liegeabschnitt 2 anschließende zusätzliche Liegeabschnitt 3 oder spiegelbildlich dazu ausgebildet. Dies gestattet einen besonders einfachen, modularen Aufbau aus vorzugsweise zumindest im Wesentlichen gleich aufgebauten Bauteilen.

Beim Darstellungsbeispiel können die insbesondere ebenen Grundplatten des Kopfabschnitts 6 und/oder des zusätzlichen Liegeabschnitts 3 durch anatomisch angepasste und/oder vorzugsweise gleichartig ausgebildete Auflagen 31 teilweise überdeckt sein, wie in Fig. 6 angedeutet, insbesondere um einen angenehmen Übergang zu dem erhöhten bzw. quer geneigten Hauptauflagebercich 9 zu bilden. Die Auflagen 31 schließen sich insbesondere an den Hauptauflagebereich 9 des Liegeabschnitts 2 auf entgegengesetzten Seiten bzw. in Längsrichtung an. Der Hauptauflagebereich 9 selbst kann insbesondere auch durch eine entsprechende Auflage oder einen sonstigen Aufbau gebildet sein.

Bei der zweiten Ausführungsform sind die weiteren Liegeabschnitte 4 und 5 vorzugsweise wahlweise an dem einen oder anderen Ende der Vorrichtung 1 bzw. wahlweise an dem zusätzlichen Liegeabschnitt 3 und dem die Kopfabstützung 6 bildenden Liegeabschnitt anbringbar. So kann je nach Bedarf die Vorrichtung 1 sehr einfach derart umgebaut werden, dass wahlweise die rechte oder die linke Mamma 12 einer Frau bzw. Patientin untersucht werden kann.

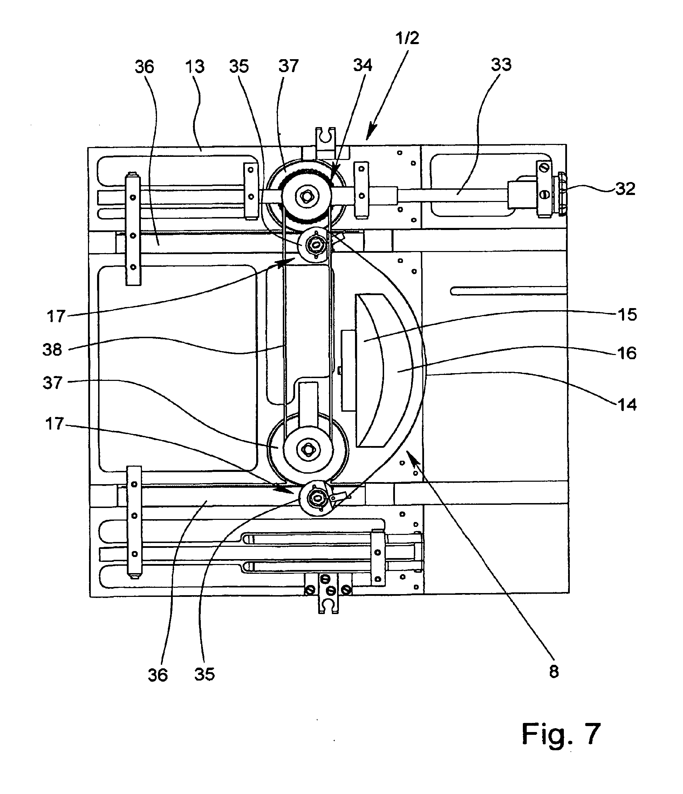

Nachfolgend wird eine zweite Ausführungsform der Komprimierungseinrichtung 8 anhand der Draufsicht gemäß Fig. 7 näher erläutert. Diese Darstellung entspricht im Wesentlichen der Darstellung gemäß Fig. 3, so dass insbesondere auch die diesbezüglichen Erläuterungen und Ausführungen vorzugsweise entsprechend oder ergänzend gelten.

Bei der zweiten Ausführungsform weist die Komprimierungseinrichtung 8 im Gegensatz zur ersten Ausführungsform vorzugsweise nur ein zum Spannen des ersten Elements 14 bzw. der Folie zu betätigendes Betätigungselement 32 anstelle der zwei Spannschrauben 19 auf. Das Betätigungselement 32 ist insbesondere radartig ausgebildet.

Das Komprimieren der Mamma 12 bzw. Spannen des ersten Elements 14 kann insbesondere von der Seite aus erfolgen, von der auch eine spätere Punktion bzw. Untersuchung der Mamma 12 erfolgt bzw. von der das Anliegen des ersten Elements 14 auf der Mamma 12 sichtbar ist. Vorzugsweise ist das Betätigungselement 32 an der genannten Seite an der Vorrichtung 1 bzw. am Liegeabschnitt 2 angeordnet, beim Darstellungsbeispiel im Bereich des Seitenauflagebereichs 10 oder darunter.

Wie bei der ersten Ausführungsform erfolgt bei der zweiten Ausführungsform das Komprimieren der Mamma 12 bzw. Spannen des ersten Elements 14 vorzugsweise manuell. Jedoch sind auch andere konstruktive Lösungen möglich.

Beim Darstellungsbeispiel ist das Betätigungselement 32 vorzugsweise über eine Welle 33 mit einem Getriebe 34, insbesondere einem Winkelgetriebe, gekoppelt, um das erste Element 14 gegen das zweite Element 15 spannen zu können.

Die Spanneinrichtung 17 weist insbesondere das Betätigungselement 32 und sonstige Elemente, wie das Getriebe 34 oder dergleichen, zum Spannen des ersten Elements 14 gegen das zweite Element 15 auf.

Bei der zweiten Ausführungsform sind die beiden Enden des ersten Elements 14 vorzugsweise wiederum schlittenartig oder gradlinig verschiebbar, um das erste Element 14 und das zweite Element 15 relativ zueinander verstellen bzw. spannen zu können. Hierzu sind die Enden des ersten Elements 14 bzw. der Folie vorzugsweise von Befestigungseinrichtungen 35 gehalten. Die Befestigungseinrichtungen 35 sind beim Darstellungsbeispiel vorzugsweise jeweils in der Art einer Spule oder Trommel oder dergleichen ausgebildet, um einen entsprechenden Endbereich des ersten Elements 14 - insbesondere mit einer vorgegebenen oder einstellbaren maximalen Spannkraft - zu halten. Hierzu können in die Befestigungseinrichtungen 35 entsprechende Rutschkupplungen oder dergleichen integriert sein, die es gestatten, dass bei Erreichen einer bestimmten Kraft das erste Element 14 - also die Folie - davon abgewickelt wird.

Die beiden Befestigungseinrichtungen 35 sind zum Spannen des ersten Elements 14 vorzugsweise längsverschiebbar geführt bzw. gehalten. Wenn - gegebenenfalls auch zuerst nur an einer Befestigungseinrichtung 35 - die maximale Kraft erreicht wird, gibt die jeweilige Befestigungseinrichtung 35 - beispielsweise durch Abwickeln - bezüglich des ersten Elements 14 nach. Auf diese Weise kann erreicht werden, dass die zu untersuchende Mamma 12 nicht übermäßig und/oder möglichst gleichmäßig komprimiert wird.

Vorzugsweise ist die Komprimierungseinrichtung 8 bzw. Spanneinrichtung 17 also derart ausgebildet, dass das erste Element 14 nur mit einer maximalen Kraft bzw. begrenzten Kraft spannbar ist und/oder ein möglichst gleichmäßiges Spannen an beiden Enden des ersten Elements 14 und/oder auf beiden Seiten der Mamma 12 erfolgt.

Es ist jedoch grundsätzlich auch möglich, dass die Befestigungseinrichtungen 35 lediglich im Wesentlichen feste bzw. unnachgiebige Widerlager oder nur beschränkt nachgiebige Widerlager für die Folie 14 bilden. Beispielsweise kann auch eine federartige Befestigung oder Widerlagerung des ersten Elements 14 im Endbereich bzw. an der jeweiligen Befestigungseinrichtung 35 erfolgen.

Die Komprimierungseinrichtung 8 bzw. Spanneinrichtung 17 bzw. jeweilige Befestigungseinrichtung 35 ist vorzugsweise derart ausgebildet, dass ein einfaches Befestigen des ersten Elements 14 beispielsweise durch Einhängen, Einstecken oder dergleichen, ermöglicht wird. Alternativ können auch die Befestigungseinrichtungen 35 mit dem ersten Element 14 zusammen auswechselbar oder von der Spanneinrichtung 17 lösbar sein.

Beim Darstellungsbeispiel sind die Befestigungseinrichtungen 35 vorzugsweise jeweils längsverschiebbar bzw. schlitzartig verschiebbar geführt, besonders bevorzugt durch eine jeweils zugeordnete Zahnstange 36. Jeder Zahnstange ist vorzugsweise ein Zahnrad 37 zugeordnet, dass mit der Zahnstange 36 kämmt. Durch Drehen des zugeordneten Zahnrads 37 ist dementsprechend die gewünschte Längsverschiebung bzw. schlittenartige Verschiebung erreichbar, wobei die Zahnstange 36 mit der zugeordneten Befestigungseinrichtung 35 zum Spannen in die eine Richtung und zum Lösen oder Entspannen des ersten Elements 14 in die andere Richtung bewegbar ist.

Beim Darstellungsbeispiel sind die beiden Befestigungseinrichtungen 35 bzw. diesen zugeordneten Zahnstangen 36 vorzugsweise synchron bzw. gleichzeitig verschiebbar. Hierzu sind beim Darstellungsbeispiel die Zahnräder 37 vorzugsweise drehgekoppelt, insbesondere über einen Riemen 38, beispielsweise einen Zahnriemen oder dergleichen. Das Betätigungselement 32 bzw. Getriebe 34 gestattet ein entsprechendes Antreiben bzw. Drehen der Zahnräder 37 und damit ein entsprechendes Verstellen der Befestigungseinrichtung 35 in Spannrichtung oder Entspannrichtung.

Die Spann- bzw. Entspannrichtung verläuft beim Darstellungsbeispiel vorzugsweise horizontal und/oder parallel zur Haupterstreckungsfläche der Vorrichtung 1. Weiter verläuft die Spann- bzw. Entspannrichtung vorzugsweise quer, insbesondere senkrecht, zur Längserstreckung der Vorrichtung 1 bzw. Längsachse einer zu untersuchenden Frau bzw. Patientin (nicht dargestellt).

Einzelne Aspekte und Merkmale der verschiedenen Ausführungsformen können beliebig miteinander kombiniert, aber auch unabhängig voneinander realisiert werden.

**Bezugszeichenliste**

| | | | |
|---|---|---|---|
| 1 | Vorrichtung | 22 | Hydraulikzylinder |
| 2 | Liegeabschnitt | 23 | Hydraulikzylinder |
| 3 | zusätzlicher Liegeabschnitt | 24 | Aufnahme |
| 4 | zusätzlicher Liegeabschnitt | 25 | Umlenkeinrichtung |
| 5 | zusätzlicher Liegeabschnitt | 26 | Kulisse |
| 6 | Kopfabstützung | 27 | Positionsarm |
| 7 | Öffnung | 28 | Gelenk |
| 8 | Komprimierungseinrichtung | 29 | Sensor |
| 9 | Hauptauflagebereich | 30 | Schlittenführung |
| 10 | Seitenauflagebereich | 31 | Auflage |
| 11 | Brustspule | 32 | Betätigungselement |
| 12 | Mamma | 33 | Welle |
| 13 | Bodenplatte | 34 | Getriebe |
| 14 | erstes Element | 35 | Befestigungseinrichtung |
| 15 | zweites Element | 36 | Zahnstange |
| 16 | Anlagefläche | 37 | Zahnrad |
| 17 | Spanneinrichtung | 38 | Riemen |
| 18 | Spannelement | | |
| 19 | Spannschraube | | |
| 20 | Positioniereinrichtung | M | Mammaerstreckungsrichtung |
| 21 | Dosierpumpe | | |

## Patentansprüche

1. Vorrichtung (1) zur Mammauntersuchung, insbesondere Biopsie, mit einer Komprimierungseinrichtung (8) zur Komprimierung einer zu untersuchenden Mamma (12), wobei die Komprimierungseinrichtung (8) ein erstes und ein zweites Element (14, 15) aufweist, wobei beide Elemente (14, 15) relativ zueinander verstellbar sind und zwischen beiden die Mamma (12) aufnehmbar und komprimierbar ist, und wobei das erste Element (14) flexibel und/oder bandartig und/oder U-förmig und/oder transparent ausgebildet ist
**dadurch gekennzeichnet,**
**dass** das zweite Element (15) eine konvexe Anlagefläche (16) für die Mamma (12) bildet, wobei die Anlagefläche (16) in einer Ebene senkrecht zur Mammaerstreckungsrichtung vom Brustansatz zur Brustspitze konvex gewölbt ist, und dass das zweite Element (15) vom ersten Element (14) weggebogen oder - geneigt ist.

2. Vorrichtung nach Anspruch 1, **dadurch gekennzeichnet, dass** das erste Element (14) folienartig ausgebildet oder aus Folie hergestellt ist.

3. Vorrichtung nach Anspruch 1 oder 2, **dadurch gekennzeichnet, dass** das erste Element (14) an einer beliebigen Stelle von einer Untersuchungseinrichtung, insbesondere einer Biopsieeinrichtung, durchdringbar, insbesondere durchstechbar, ist.

4. Vorrichtung nach einem der voranstehenden Ansprüche, **dadurch gekennzeichnet, dass** die Komprimierungseinrichtung (8) eine Spanneinrichtung (17) zum Spannen des ersten Elements (14) aufweist, wobei das erste Element (14) an zwei entgegengesetzten Seiten oder Enden bzw. Endbereichen spannbar oder translatorisch verschiebbar ist.

5. Vorrichtung nach einem der voranstehenden Ansprüche, **dadurch gekennzeichnet, dass** die Komprimierungseinrichtung (8) derart ausgebildet ist, dass das erste Element (14) nur mit einer begrenzten Kraft gegen die am zweiten Element (15) wiedergelagerte Mamma (12) spannbar ist.

6. Vorrichtung nach einem der voranstehenden Ansprüche, **dadurch gekennzeichnet, dass** das erste Element (14) das zweite Element (15) zumindest im Wesentlichen U-förmig oder halbkreisförmig umgibt.

7. Vorrichtung nach einem der voranstehenden Ansprüche, **dadurch gekennzeichnet, dass** das erste Element (14) auswechselbar und/oder einhängbar von einer Spanneinrichtung (17) der Komprimierungseinrichtung (8) gehalten ist.

8. Vorrichtung nach einem der voranstehenden Ansprüche, **dadurch gekennzeichnet, dass** das erste Element (14) dünn und/oder streifenartig ausgebildet ist.

9. Vorrichtung nach einem der voranstehenden Ansprüche, **dadurch gekennzeichnet, dass** die Komprimierungseinrichtung (8) zum Verstellen der beiden Elemente relativ zueinander von der Seite her betätigbar ist, an der das erste Element (14) an der Mamma (12) anliegt.

10. Vorrichtung nach einem der voranstehenden Ansprüche, **dadurch gekennzeichnet, dass** die Anlagefläche (16) in einer Ebene parallel zur Mammaerstreckungsrichtung (vom Brustansatz zur Brustspitze) konvex gewölbt ist.

11. Vorrichtung nach einem der voranstehenden Ansprüche, **dadurch gekennzeichnet, dass** die Anlagefläche (16) in einer Ebene parallel zur Mammaerstreckungsrichtung (M) und/oder ausgehend von einem Bereich, der der Brustspitze benachbart ist, hin zu einem Bereich, der dem Brustansatz benachbart ist, vom ersten Element (14) weggebogen oder -geneigt ist.

## Claims

1. A device (1) for a mammary examination, in particular a biopsy, having a compression device (8) for the compression of a mamma to be examined (12), wherein the compression device (8) has a first and a second element (14, 15), wherein both elements can be adjusted relative to each other and the mamma (12) is receivable and compressible between both elements, and wherein the first element (14) is designed to be flexible and/or ribbon-like and/or U-shaped and/or transparent,
**characterized in**
**that** the second element (15) forms a convex contact surface (16) for the mamma (12), wherein the contact surface (16) is convexly curved in a plane perpendicular to the mamma extension direction from the breast base to the breast tip, and that the second element (15) is bent away or tilted away from the first element (14).

2. A device according to claim 1, **characterized in that** the first element (14) is designed film-like or is produced from film.

3. A device according to claim 1 or 2, **characterized in that** the first element (14) is penetrable, in particular can be pierced at any position by an examination device, in particular a biopsy device.

4. A device according to one of the preceding claims, **characterized in that** the compression device (8) has a tension device (17) for tensioning the first element (14), wherein the first element (14) is tensible or movable translationally at two opposite sides or ends or end areas.

5. A device according to one of the preceding claims, **characterized in that** the compression device (8) is designed in such a manner that the first element (14) is only tensible with a limited force against the mamma (12) remounted on the second element (15).

6. A device according to one of the preceding claims, **characterized in that** the first element (14) encompasses the second element (15) at least substantially in a U-shaped or semi-circular manner.

7. A device according to one of the preceding claims, **characterized in that** the first element (14) is kept demountable and/or mountable by a tension device (17) of the compression device (8).

8. A device according to one of the preceding claims, **characterized in that** the first element (14) is designed thin and/or strip-like.

9. A device according to one of the preceding claims, **characterized in that** the compression device (8) is operable for the adjustment of the two elements relative to each other from the side, on which the first element (14) is in contact with the mamma (12).

10. A device according to one of the preceding claims, **characterized in that** the contact surface (16) is convexly curved in a plane parallel to the mamma extension direction (from the breast base to the breast tip).

11. A device according to one of the preceding claims, **characterized in that** the contact surface (16) is bent away or tilted away from the first element (14) in a plane parallel to the mamma extension direction (M) and /or originating from an area, which is adjacent to the breast tip, towards an area, which is adjacent to the top breast base.

## Revendications

1. Dispositif (1) pour l'examen du sein, plus particulièrement une biopsie, avec un dispositif de compression (8) pour la compression d'un sein (12) à examiner, le dispositif de compression (8) comprend un premier et un deuxième élément (14, 15), ces deux éléments (14, 15) étant réglables l'un par rapport à l'autre et le sein (12) pouvant être logé et comprimé entre eux, et le premier élément (14) étant flexible et/ou présentant la forme d'une bande et/ou présentant une forme de U et/ou étant transparent,
**caractérisé**
**en ce que** le deuxième élément (15) forme une surface d'appui convexe (16) pour le sein (12), la surface d'appui (16) étant incurvée de manière convexe dans un plan perpendiculaire au sens d'extension du sein, de la base à la pointe du sein, et en ce que le deuxième élément (15) est plié ou incliné loin du premier élément (14).

2. Dispositif selon la revendication 1, **caractérisé en ce que** le premier élément (14) est conçu sous la forme d'un film ou est fabriqué à partir d'un film.

3. Dispositif selon la revendication 1 ou 2, **caractérisé en ce que** le premier élément (14) peut être pénétré, plus particulièrement perforé, à un endroit quelconque par un dispositif d'examen, plus particulièrement un dispositif de biopsie.

4. Dispositif selon l'une des revendications précédentes, **caractérisé en ce que** le dispositif de compression (8) comprend un dispositif de serrage (17) pour le serrage du premier élément (14), le premier élément (14) pouvant être serré ou coulissé en translation sur deux côtés, ou extrémités ou zones d'extrémités opposées.

5. Dispositif selon l'une des revendications précédentes, **caractérisé en ce que** le dispositif de compression (8) est conçu de façon à ce que le premier élément (14) peut être serré uniquement avec une force limitée contre le sein (12) appuyé sur le deuxième élément (15).

6. Dispositif selon l'une des revendications précédentes, **caractérisé en ce que** le premier élément (14) entoure le deuxième élément (15) au moins globalement en forme de U ou en forme d'arc de cercle.

7. Dispositif selon l'une des revendications précédentes, **caractérisé en ce que** le premier élément (14) est maintenu de manière amovible et/ou accrochable par un dispositif de serrage (17) du dispositif de compression (8).

8. Dispositif selon l'une des revendications précédentes, **caractérisé en ce que** le premier élément (14) est mince et/ou présente la forme d'une bande.

9. Dispositif selon l'une des revendications précédentes, **caractérisé en ce que** le dispositif de compression (8) est actionnable pour le réglage des deux éléments l'un par rapport à l'autre du côté où le premier élément (14) s'appuie contre le sein (12).

10. Dispositif selon l'une des revendications précédentes, **caractérisé en ce que** la surface d'appui (16) est incurvée de manière convexe dans un plan parallèle au sens d'extension du sein (de la base à la pointe du sein).

11. Dispositif selon l'une des revendications précédentes, **caractérisé en ce que** la surface d'appui (16) est pliée ou inclinée loin du premier élément (14), dans un plan parallèle au sens d'extension du sein (M) et/ou à partir d'une zone voisine de la pointe de la poitrine en direction d'une zone voisine de la base de la poitrine.
